# EUROPEAN PATENT SPECIFICATION

(11) **EP 2 618 151 B1**
(45) Date of publication and mention of the grant of the patent: **20.07.2016**
(21) Application number: 13151783.1
(22) Date of filing: 18.01.2013
(51) Int. Cl.: G01N 33/543, G01N 33/558

(54) **Low volume assay device having increased sensitivity**
Niedermengen-Analysevorrichtung mit gesteigerter Empfindlichkeit
Dispositif de dosage de faible volume ayant une sensibilité accrue

(30) Priority: 20.01.2012 US 201261588758 P
(43) Date of publication of application: 24.07.2013
(73) Proprietor: ORTHO-CLINICAL DIAGNOSTICS, INC., Raritan, NJ 08869 (US)
(72) Inventor: Hosimer, Philip C., New York, NY 14617 (US); Ding, Zhong, New York, NY 14534 (US); Heavner, David A., New York, NY 14450 (US); Scalice, Edward R., New York, NY 14526 (US); Danielson, Susan, New York, NY 14472 (US); Kanaley, James D., New York, NY 14472 (US); Tomasso, David A., New York, NY 14626 (US); Warren, Timothy C., New York, NY 14626 (US)
(74) Representative: Bergenstråhle Group AB

(56) References cited:
- WO-A1-99/17100
- WO-A1-2006/137785
- WO-A2-03/048736
- US-B1- 6 228 660

## Description

### Field of the Invention

The present invention relates to the field of diagnostic assays, and in particular to lateral flow assays where an analyte to be detected is present in a biological or non-biological sample.

### Background

Diagnostic assays are widespread and central for the diagnosis, treatment and management of many diseases. Different types of diagnostic assays have been developed over the years in order to simplify the detection of various analytes in clinical samples such as blood, serum, plasma, urine, saliva, tissue biopsies, stool, sputum, skin or throat swabs and tissue samples or processed tissue samples. These assays are frequently expected to give a fast and reliable result, while being easy to use and inexpensive to manufacture. Understandably it is difficult to meet all these requirements in one and the same assay. In practice, many assays are limited by their speed. Another important parameter is sensitivity. Recent developments in assay technology have led to increasingly more sensitive tests that allow detection of an analyte in trace quantities as well the detection of disease indicators in a sample at the earliest time possible.

A common type of disposable assay device includes a zone or area for receiving the liquid sample, a conjugate zone also known as a reagent zone, and a reaction zone also known as a detection zone. These assay devices are commonly known as lateral flow test strips. They employ a porous material, e.g., nitrocellulose, defining a path for fluid flow capable of supporting capillary flow. Examples include those shown in US Patent Nos. 5,559,041, 5,714,389, 5,120,643, and 6,228,660.

The sample-addition zone frequently consists of a more porous material, capable of absorbing the sample, and, when separation of blood cells is desired, also effective to trap the red blood cells. Examples of such materials are fibrous materials, such as paper, fleece, gel or tissue, comprising e.g. cellulose, wool, glass fiber, asbestos, synthetic fibers, polymers, or mixtures of the same.

Another type of assay device is a non-porous assay having projections to induce capillary flow. Examples of such assay devices include the open lateral flow device as disclosed in WO 2003/103835, WO 2005/089082, WO 2005/118139, and WO 2006/137785.

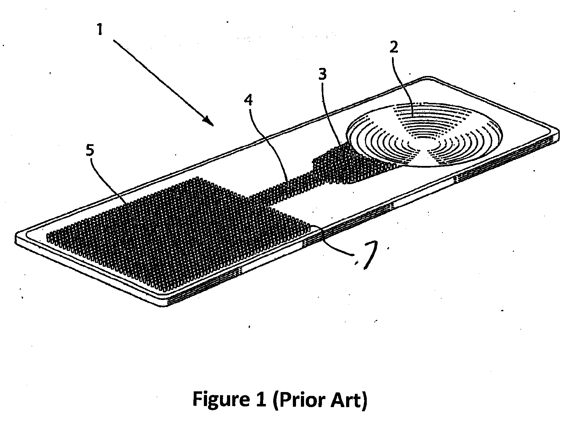

A known non-porous assay device is shown in Figure 1. The assay device 1, has at least one sample addition zone 2, a reagent zone 3, at least one detection zone 4, and at least one wicking zone 5. The zones form a flow path by which sample flows from the sample addition zone to the wicking zone. Also included are capture elements, such as antibodies, in the detection zone 4, capable of binding to the analyte, optionally deposited on the device (such as by coating); and a labeled conjugate material also capable of participating in reactions that will enable determination of the concentration of the analyte, deposited on the device in the reagent zone, wherein the labeled conjugate material carries a label for detection in the detection zone.

The conjugate material is dissolved as the sample flows through the reagent zone forming a conjugate plume of dissolved labeled conjugate material and sample that flows downstream to the detection zone. In some types of devices the conjugate material is deposited in the center of the conjugate zone and is dissolved from the sides as sample and/or a separate wash is flowing past the deposited conjugate material along the side edges. An example of such a deposited conjugate material is shown in Figure 1 of US2009-0311805A1. As the conjugate plume flows into the detection zone, the conjugated material will be captured by the capture elements such as via a complex of conjugated material and analyte (as in a "sandwich" assay) or directly (as in a "competitive" assay). Unbound dissolved conjugate material will be swept past the detection zone into the wicking zone 5.

An instrument such as that disclosed US 20060289787A1, US20070231883A1, US 7,416,700 and US 6,139,800, is able to detect the bound conjugated material in the reaction zone. Common labels include fluorescent dyes that can be detected by instruments which excite the fluorescent dyes and incorporate a detector capable of detecting the resulting fluorescence. Such instruments have a read window that has a width that is typically on the order of 1mm, which is a generally sufficient width to read enough signal, subject to an adequate width of the conjugate plume.

One drawback with such known assay devices such as those described above, is that the dissolved conjugate stream in the reaction zone is often narrower than the read window of the instrument, which may negatively impact assay sensitivity and variability. This is of particular concern for designs such as those described above where the conjugate material is deposited in the center of the conjugate zone and is dissolved from the sides as sample is flowing past. If the channel is made wider than the read window, although the dissolved conjugate width may match the read window size, the fluid sample outside the read window contributes no signal and is wasted. This is also of particular concern for a smaller sample volume design described below. Throughout the remainder of the description the term "smaller sample volume" or "smaller volume" design is used interchangeably with "miniaturized" design.

The sample size for such typical assay devices as shown in Figure 1 are generally on the order of 200µl of whole blood. Such a sample size requires a venous blood draw from a medical professional such as a phlebotomist. There is an increasing need for lateral flow devices that are able to function with a much smaller sample size to accommodate the amount of blood available from a so-called "fingerstick" blood draw, which is on the order of 25 µl or less. Such a small amount of sample is the amount of blood in a drop of blood after pricking a finger tip with a lancet. Home blood glucose meters typically use a drop of blood obtained in such a fashion to provide glucose levels in blood. Such a smaller sample size would not require a medical professional to draw the blood and would provide greater comfort to the patients providing the sample for analysis.

To reduce the sample size required, the dimensions of the lateral flow assay devices are reduced to accommodate the smaller sample size. However, it has been found that reducing the sample size and dimensions of the device provides inadequate conjugate material in the reaction zone and accordingly less signal that can be read by the instrument, in some instances up to a 5x lower signal and poor sensitivity. The inadequate conjugate material in the reaction zone is believed to be due to reduced sample size and inefficient use of the sample in the device, amongst other conditions. Another drawback of reducing dimensions is the width of the reaction zone will also be reduced, again making less signal available that can be read by the instrument. Also, it has been found that a smaller device has reduced flow time and conjugate material contact time, resulting in less binding between the analyte in the sample and the conjugate material in the case of a sandwich-type assay.

The problems of a conjugate plume not covering as much of the width of the reaction zone as possible is a particular problem in smaller volume devices that have a narrower reaction zone, and in those devices where the deposited conjugate material is dissolved from the sides. In other words, it is important for the conjugate plume to spread across as much of the width of the reaction zone as possible to provide the maximum amount of signal to be read by the read window of the instrument.

Accordingly, there is a need for an assay device that can recover the loss of signal that occurs from reducing sample size in a smaller volume assay device. There is also a need for an assay device that can provide a wider conjugate plume in the detection zone, better mix the dissolved conjugate and sample, and make more efficient use of sample in an assay device, particularly in those devices where the conjugate material is deposited in the center of the conjugate zone and is dissolved from the sides.

### Summary of the Invention

The present invention is directed to an assay device that alleviates one or more the foregoing problems described above.

One aspect of the invention is directed to an assay device that includes: a liquid sample zone; a reagent zone downstream and in fluid communication with the sample addition zone containing a reagent material; a detection zone in fluid communication with the reagent zone, wherein the detection zone has a substrate and projections which extend substantially vertically from the substrate, wherein the projections have a height, cross-section and a distance between one another that defines a capillary space between the projections capable of generating capillary flow parallel to the substrate surface; wherein the reagent zone comprises at least two reagent cells containing the reagent material and arranged in the reagent zone such that each reagent cell experiences the same flow conditions of sample from the sample addition zone, wherein the reagent cells divide the sample flow from the sample addition zone into multiple flow streams, and one or more flow control elements disposed downstream from the reagent zone which combine the multiple flow streams into fewer flow streams; wherein the device is capable of creating a reagent plume in the detection zone that comprises liquid sample and dissolved reagent, wherein the width of the reagent plume extends across at least 80 % of the width of the detection zone; and a wicking zone in fluid communication with the detection zone having a capacity to receive liquid sample flowing from the detection zone.

According to another aspect of the invention, there has been provided a method for performing an assay on a liquid sample for the detection of one or more analytes of interest. The method includes the steps of: providing a liquid sample addition zone for receiving the liquid sample; providing a reagent zone in fluid communication with the sample addition zone, said reagent zone comprising at least two reagent cells containing a reagent material and arranged in the reagent zone such that each reagent cell experiences the same flow conditions of sample from said sample addition zone, wherein the reagent cells divide the sample flow from the sample addition zone into multiple flow streams; and providing a detection zone in fluid communication with the reagent zone having capture elements bound thereto, wherein the detection zone has a substrate and projections which extend substantially vertically from the substrate, wherein the projections have a height, cross-section and a distance between one another that defines a capillary space between the projections capable of generating capillary flow parallel to the substrate surface; providing a wicking zone in fluid communication with the detection zone having a capacity to receive liquid sample flowing from the detection zone; dispensing the sample onto the sample zone, whereby the sample flows by capillary action from the sample zone and into the reagent zone, where the sample dissolves the reagent material and forms a reagent plume that comprises liquid sample and dissolved reagent; flowing the sample/reagent plume by capillary action into the detection zone, wherein the width of the reagent plume extends across at least 80 % of the width of the detection zone, wherein a signal representative of the presence of concentration of analyte(s) or control(s) is produced; and reading the signal that is produced in the detection zone to determine the presence or concentration of the one or more analyte.

Further objects, features and advantages of the present invention will be apparent to those skilled in the art from detailed consideration of the preferred embodiments that follow.

### Brief Description of the Drawings

**Figure 1** shows a known assay device.
**Figure 2** shows a schematic view of an assay device according to one embodiment of the present invention.
**Figure 3** shows a schematic view of an assay according to another embodiment of the invention.
**Figure 4** shows a schematic view of an assay according to another embodiment of the invention having multiple reagent elements.
**Figure 5** shows a schematic view of an assay according to another embodiment of the invention having multiple reagent elements.
**Figures 6** **and** **7** show sensitivity of different assay device designs with NTproBNP as the analyte.
**Figure 8** shows a comparison of conjugate dissolution time for an assay devices having a wider detection zone compared to an assay device having a narrower detection zone.
**Figures 9A-D** are photos showing the width of a reagent plume from a multiple reagent zone according to the present invention compared to a single reagent zone.
**Figure 10** is a plot of procalcitonin concentration vs. mean peak area using a whole blood sample and a wash.
**Figure 11** is a plot of procalcitonin concentration vs. mean peak area using a whole blood sample.

### Detailed Description of Preferred Embodiments

As used in this specification and the appended claims, the singular forms "a", "an" and "the" include plural referents unless the context clearly dictates otherwise.

The term "about" as used in connection with a numerical value throughout the description and the claims denotes an interval of accuracy, familiar and acceptable to a person skilled in the art. The interval is preferably ± 10 %.

The term "sample" herein means a volume of a liquid, solution or suspension, intended to be subjected to qualitative or quantitative determination of any of its properties, such as the presence or absence of a component, the concentration of a component, etc. Typical samples in the context of the present invention are human or animal bodily fluids such as blood, plasma, serum, lymph, urine, saliva, semen, amniotic fluid, gastric fluid, phlegm, sputum, mucus, tears, stool, etc. Other types of samples are derived from human or animal tissue samples where the tissue sample has been processed into a liquid, solution, or suspension to reveal particular tissue components for examination. The embodiments of the present invention are applicable to all bodily samples, but preferably to samples of whole blood, urine or sputum.

In other instances, the sample can be related to food testing, environmental testing, bio-threat or bio-hazard testing, etc. This is only a small example of samples that can be used in the present invention.

In the present invention, the determination based on lateral flow of a sample and the interaction of components present in the sample with reagents present in the device or added to the device during the procedure and detection of such interaction, either qualitatively or quantitatively, may be for any purpose, such as diagnostic purposes. Such tests are often referred to as lateral flow assays.

Examples of diagnostic determinations include, but are not limited to, the determination of analytes, also called markers, specific for different disorders, e.g. chronic metabolic disorders, such as blood glucose, blood ketones, urine glucose (diabetes), blood cholesterol (atherosclerosis, obesity, etc); markers of other specific diseases, e.g. acute diseases, such as coronary infarct markers (e.g. troponin-T, NT-ProBNP), markers of thyroid function (e.g. determination of thyroid stimulating hormone (TSH)), markers of viral infections (the use of lateral flow immunoassays for the detection of specific viral antibodies); etc.

Yet another important field is the field of companion diagnostics where a therapeutic agent, such as a drug, is administered to an individual in need of such a drug. An appropriate assay is then conducted to determine the level of an appropriate marker to determine whether the drug is having its desired effect. Alternatively, the assay device of the present invention can be used prior to administration of a therapeutic agent to determine if the agent will help the individual in need.

Yet another important field is that of drug tests, for easy and rapid detection of drugs and drug metabolites indicating drug abuse; such as the determination of specific drugs and drug metabolites (e.g. THC) in urine samples etc.

The term "analyte" is used as a synonym of the term "marker" and intended to encompass any chemical or biological substance that is measured quantitatively or qualitatively and can include small molecules, proteins, antibodies, DNA, RNA, nucleic acids, virus components or intact viruses, bacteria components or intact bacteria, cellular components or intact cells and complexes and derivatives thereof.

The terms "zone", "area" and "site" are used in the context of this description, examples and claims to define parts of the fluid flow path on a substrate, either in prior art devices or in a device according to an embodiment of the invention.

The term "reaction" is used to define any reaction, which takes place between components of a sample and at least one reagent or reagents on or in the substrate, or between two or more components present in the sample. The term "reaction" is in particular used to define the reaction, taking place between an analyte and a reagent as part of the qualitative or quantitative determination of the analyte.

The term "substrate" means the carrier or matrix to which a sample is added, and on or in which the determination is performed, or where the reaction between analyte and reagent takes place.

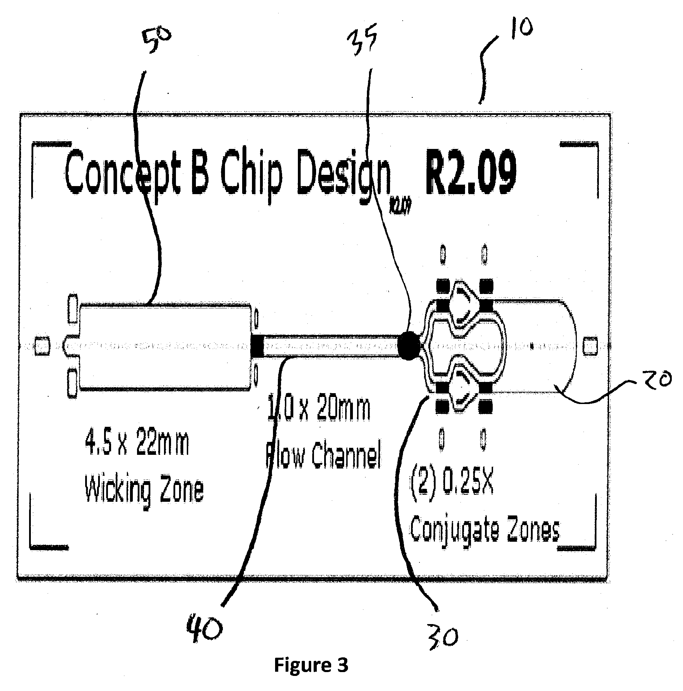

The present invention is directed to a lateral flow assay device as defined in claim 1 for determining the presence or amount of at least one analyte that solves, at least in part, the problem of lowered signal due to a narrower reagent plume (described below) that may result from the conjugate material being dissolved from the sides, or reduced sample size that is used in a miniaturized assay device. Figure3 shows a schematic view of an embodiment of a device according to the invention. The assay device 10, has at least one sample zone 20, at least one reagent zone 30, at least one detection zone 40, and at least one wicking zone 50. The zones form a flow path by which sample flows from the sample addition zone to the wicking zone.

In order to achieve the desired goal of reducing the amount of sample required, the present inventors discovered that simply scaling down a conventionally sized device was insufficient because, as noted above, it resulted in insufficient signal being read by the instrument. Upon further investigation, it was discovered that in a conventionally sized assay device, i.e., one that uses on the order of 200µl of blood, only about 10% of the analyte in the sample is captured and detected in the detection zone. While this may be a sufficient efficiency for larger sample sizes, such a low efficiency will result in insufficient signal for devices of the present invention that have significantly smaller dimensions and substantially less sample as compared to conventional devices.

In order to maximize analyte capture in a lower volume device and sample size, the present inventors found, after extensive research, that modifications were required in order to provide a miniaturized device having adequate signal. Briefly, these include:
Increasing the effective area of the detection zone by increasing the width of the dissolved reagent plume coming from the reagent zone and increasing the width of the flow channel;
Increasing total assay flow time, to both increase the contact time between the reagent material and analyte in the reagent zone, and to increase contact time between labeled analyte and capture elements in the detection zone; and
Ensuring that the reagent dissolution time in the reagent zone is maximized, while still allowing adequate wash time during the final minutes of the assay flow time. These modifications are described in more detail below.

Components of the assay device (i.e., a physical structure of the device whether or not a discrete piece from other parts of the device) can be prepared from copolymers, blends, laminates, metalized foils, metalized films or metals. Alternatively, device components can be prepared from copolymers, blends, laminates, metalized foils, metalized films or metals deposited one of the following materials: polyolefins, polyesters, styrene containing polymers, polycarbonate, acrylic polymers, chlorine containing polymers, acetal homopolymers and copolymers, cellulosics and their esters, cellulose nitrate, fluorine containing polymers, polyamides, polyimides, polymethylmethacrylates, sulfur containing polymers, polyurethanes, silicon containing polymers, glass, and ceramic materials. Alternatively, components of the device are made with a plastic, elastomer, latex, silicon chip, or metal; the elastomer can comprise polyethylene, polypropylene, polystyrene, polyacrylates, silicon elastomers, or latex. Alternatively, components of the device can be prepared from latex, polystyrene latex or hydrophobic polymers; the hydrophobic polymer can comprise polypropylene, polyethylene, or polyester. Alternatively, components of the device can comprise TEFLON@, polystyrene, polyacrylate, or polycarbonate. Alternatively, device components are made from plastics which are capable of being embossed, milled or injection molded or from surfaces of copper, silver and gold films upon which may be adsorbed various long chain alkanethiols. The structures of plastic which are capable of being milled or injection molded can comprise a polystyrene, a polycarbonate, or a polyacrylate. In a particularly preferred embodiment, the assay device is injection molded from a cyclic olefin polymer, such as those sold under the name Zeonor®. Preferred injection molding techniques are described in U.S. Patent Nos. 6,372,542, 6,733,682, 6,811,736, 6,884,370, and 6,733,682.

The flow path can include open or closed paths, grooves, and capillaries. Preferably the flow path comprises a lateral flow path of adjacent projections, having a size, shape and mutual spacing such that capillary flow is sustained through the flow path. In one embodiment, the flow path is in a channel within the substrate having a bottom surface and side walls. In this embodiment, the projections protrude from the bottom surface of the channel. The side walls may or may not contribute to the capillary action of the liquid. If the sidewalls do not contribute to the capillary action of the liquid, then a gap can be provided between the outermost projections and the sidewalls to keep the liquid contained in the flow path defined by the projections. Figure 1 shows projections 7.

In one embodiment the flow path is at least partially open. In another embodiment the flow path is entirely open. Open means that there is no lid or cover at a capillary distance. Thus the lid, if present as a physical protection for the flow path, does not contribute to the capillary flow in the flow path. An open lateral flow path is described for example in the following published applications: WO 2003/103835, WO 2005/089082; WO 2005/118139; WO 2006/137785; and WO 2007/149042. The projections have a height (H), diameter (D) and a distance or distances between the projections (t1, t2) such, that lateral capillary flow of the fluid, such as plasma, preferably human plasma, in the zone is achieved. These dimensions are shown in US 2006/0285996. In addition to optimizing the above-mentioned height, diameter and a distance or distances between the projections, the projections may be given a desired chemical, biological or physical functionality, e.g. by modifying the surface of the projections. In one embodiment, the projections have a height in the interval of about 15 to about 150 µm, preferably about 30 to about 100 µm, a diameter of about 10 to about 160 µm, preferably 40 to about 100 µm, and a gap or gaps between the projections of about 3 to about 200 µm, preferably 5 to 50 µm or 10 to about 50 µm from each other. The flow channel may have a length of about 5 to about 500 mm, preferably about 10 to about 100 mm, and a width of about 0.3 to about 10 mm, preferably about 0.3 to about 3 mm, preferably about 0.5 to 1.5, and preferably about 0.5 to 1.2 mm.

While most detection will occur in the detection zone portion of the fluid flow path, it is also possible that detection may occur in other parts of the device. For example, non-invasive, non-reactive sample integrity measurements may occur between the sample zone and the reagent zone or reagent addition zone, preferably after a filter element, if present. Other measurements may include blanks reads, one part of a two part reaction sequence as for measuring both hemoglobin and glycated hemoglobin for determination of HbA1c, etc.

The liquid sample zone 20, also referred to as the liquid sample addition zone, receives sample from a sample dispenser, such as a pipette. The sample is typically deposited onto the top of the zone. The sample addition zone is capable of transporting the liquid sample from the point where the sample is deposited to the reagent zone, through an optional filter and reagent addition zone, preferably through capillary flow. The capillary flow inducing structure can include porous materials, such as nitrocellulose, or preferably through projections, such as micro-pillars, as shown in Figure 1. In those devices that can use finger stick volumes of blood, the sample can be directly touched off from the finger, or by a capillary pipette such as described in co pending application.

A filter material (not shown) can be placed in the sample addition zone to filter particulates from the sample or to filter blood cells from blood so that plasma can travel further through the device.

Located between the sample addition zone and the detection zone is a reagent zone 30. The reagent zone can include reagent(s) integrated into the analytical element and are generally reagents useful in the reaction---binding partners such as antibodies or antigens for immunoassays, substrates for enzyme assays, probes for molecular diagnostic assays, or are auxiliary materials such as materials that stabilize the integrated reagents, materials that suppress interfering reactions, etc. Generally one of the reagents useful in the reaction bears a detectable signal as discussed below. In some cases the reagents may react with the analyte directly or through a cascade of reactions to form a detectable signal such as, but not restricted to, a molecule detectable using spectroscopy such as a colored or fluorescent molecule. The amount of reagent in the reagent zone can be adjusted by the length of reagent deposited into the device while maintaining the same reagent width. The amount of reagent can also be adjusted by changing the width while maintaining the length. The amount of reagent can further be adjusted by changing both width and length simultaneously. In one preferred embodiment, the reagent zone includes conjugate material. The term conjugate means any moiety bearing both a detection element and a binding partner.

The detection element is an agent which is detectable with respect to its physical distribution or/and the intensity of the signal it delivers, such as but not limited to luminescent molecules (e.g. fluorescent agents, phosphorescent agents, chemiluminescent agents, bioluminescent agents and the like), colored molecules, molecules producing colors upon reaction, enzymes, radioisotopes, ligands exhibiting specific binding and the like. The detection element also referred to as a label is preferably chosen from chromophores, fluorophores, radioactive labels, and enzymes. Suitable labels are available from commercial suppliers, providing a wide range of dyes for the labeling of antibodies, proteins, and nucleic acids. There are, for example, fluorophores spanning practically the entire visible and infrared spectrum. Suitable fluorescent or phosphorescent labels include for instance, but are not limited to, fluoresceins, Cy3, Cy5 and the like. Suitable chemoluminescent labels are for instance but are not limited to luminol, cyalume and the like.

Similarly, radioactive labels are commercially available, or detection elements can be synthesized so that they incorporate a radioactive label. Suitable radioactive labels are for instance but are not limited to radioactive iodine and phosphorus; e.g. ¹²⁵I and ³²P.

Suitable enzymatic labels are, for instance, but are not limited to, horseradish peroxidase, beta-galactosidase, luciferase, alkaline phosphatase and the like. Two labels are "distinguishable" when they can be individually detected and preferably quantified simultaneously, without significantly disturbing, interfering or quenching each other. Two or more labels may be used, for example, when multiple analytes or markers are being detected.

The binding partner is a material that can form a complex that can be used to determine the presence of or amount of an analyte. For example, in an "sandwich" assay, the binding partner in the conjugate can form a complex including the analyte and the conjugate and that complex can further bind to another binding partner, also called a capture element, integrated into the detection zone. In a competitive immunoassay, the analyte will interfere with binding of the binding partner in the conjugate to another binding partner, also called a capture element, integrated into the detection zone. Example binding partners included in conjugates include antibodies, antigens, analyte or analyte-mimics, protein, etc.

As described above, the inventors found that to increase the amount of signal that can be detected by the instrument in the detection zone, the reagent plume from the reagent zone had to be as wide as possible to cover as much of the width of the flow path through the detection zone as possible. Any technique that can increase the width of the reagent plume can be used in the present invention.

One preferred embodiment for increasing the width of the reagent plume is described in co-pending application entitled "Assay Device Having Multiple Reagent Cells" (Application No. 61/588738, Attorney Docket No. CDS 5104USPSP, first named inventor: Zhong Ding), filed January 20, 2012. In summary, multiple areas having reagent material (hereinafter referred to as "reagent cells") in a reagent zone along with elements to recombine multiple flow streams that result from the multiple reagent cells into one flow stream results in a more desirably mixed, wider reagent plume as it leaves the reagent zone and enters the detection zone.

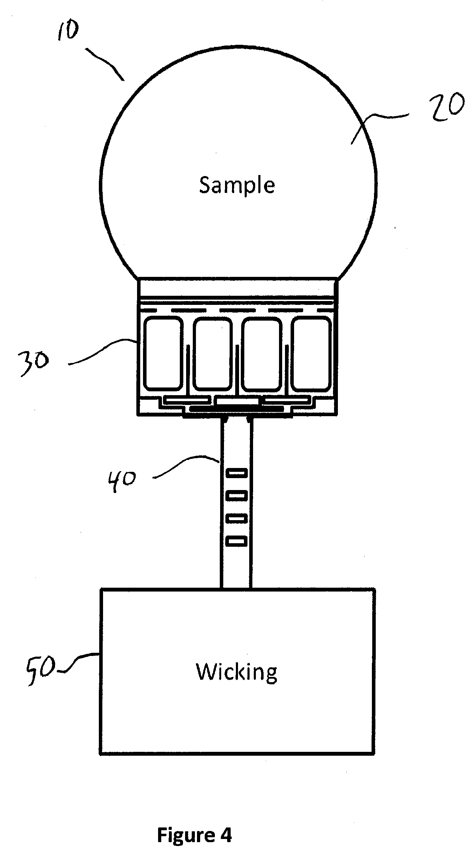

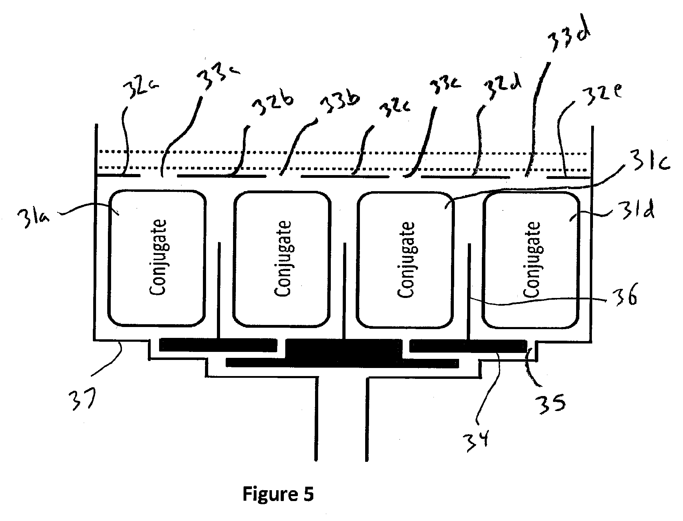

In one preferred embodiment shown in Figure 4 and in more detail in Figure 5 multiple and preferably identical reagent cells, i.e., greater than two cells (4 cells in the case of Figures 4 and 5) are arranged in a way such that each one reagent cell experiences the same flow conditions due to the symmetry of the geometry of the cells in the reagent zone. The dissolved reagents from each reagent cell flows through channel gates formed by flow control elements, and merge to form a single stream as it flows to the detection zone.

More specifically Figures 4 and 5 show an embodiment that contains four reagent cells 31 a-d. The reagent cells are symmetrically arranged in the reagent zone, such that each will experience substantially identical flow conditions of sample as the sample stream passes out of the sample addition zone into the reagent zone. While any number of reagent cells two or greater (e.g., 3, 4, 5, 6, 7, etc.) are within the scope of the present invention, it is preferred to have an geometric progression of cells represented by the formula, **2ⁿ** reagent cells, where n is a non-zero, non-negative integer. Thus, the number of cells in this preferred embodiment would be 2, 4, 8, 16, etc. reagent cells. For example, for 8 cells, n would be 3 and for 16 cells, n would be 4, etc.

Located downstream from the reagent zone are downstream flow control elements 34 and flow channel gates 35, which are arranged to combine the multiple streams coming off of the reagent cells back into a single stream for transport to the detection zone. The downstream flow control elements combine the multiple streams into a smaller number of streams until a single flow stream is achieved.

In a preferred embodiment, flow control elements 34 will have a portion 36 that extends between each of the reagent cells in the direction of fluid flow as shown in Figures 3. In this embodiment, **2ⁿ** reagent cells will result in (2ⁿ)x2 flow streams. A first stage set of flow control elements will define 2ⁿ first stage of flow control gates immediately downstream of the reagent cell and centered along the axis of symmetry of the reagent cell in the direction of flow. Exiting the first stage of flow control gates will be 2ⁿ flow streams. A second stage of flow control elements will define 2⁽ⁿ⁻¹⁾ flow control gates, which results in 2⁽ⁿ⁻¹⁾ flow streams, and so on until a single flow stream results. The resulting single flow stream will have a wider reagent plume than would have been possible with known lateral flow devices, resulting in more signal.

The flow control elements can be any structure that redirects flow either before or after the reagent cells. They can be structures protruding from the substrate of the assay device and are formed in the same manner as the micro posts described above. Some of the structures can be sidewalls of the flow channels where the flow channels narrow as shown by reference numeral 37 in Figure 5.

By using multiple reagent cells in the reagent zone, the width of the dissolved reagent plume entering the detection zone can be well defined and controlled by the number of reagent cells and the flow channels. The increased number of cells increases the width of the reagent stream. The much larger surface to volume ratio made possible by multiple reagent cells allows better reagent dissolution due to larger wetting area per unit volume, given the same flow rate. In addition, the flow rate at each cell can be lower while maintaining the overall flow rate at the desired level. For example, for a four reagent cell design, the flow rate past each of the reagent cell will be ¼ of the original flow rate entering the reagent cell. This provides more time for the sample to interact with the reagent material in the reagent cell, increasing dissolution of the reagent material into the sample flow.

Another advantage of the multiple reagent cell design is that the longer and narrower flow path with bends provided by the flow control elements, provides better mixing by both diffusion and convection.

Another embodiment for increasing the width of the reagent plume is to deposit reagent material in the reagent zone across the entire width of the reagent zone flow path. The height of the deposited reagent material is lower than is used when the material is deposited only in part of the width of the reagent zone flow path. As the sample flows from the sample addition zone, it will encounter deposited reagent material across the entire width of the reagent zone. The sample will flow over the top of the reagent material. Since the sample fluid cannot avoid contact with the reagent material, all of the sample will dissolve and mix with the reagent material. As a result, the reagent plume will exit the reagent zone covering a significant, in not the entire, width of the reagent zone flow path.

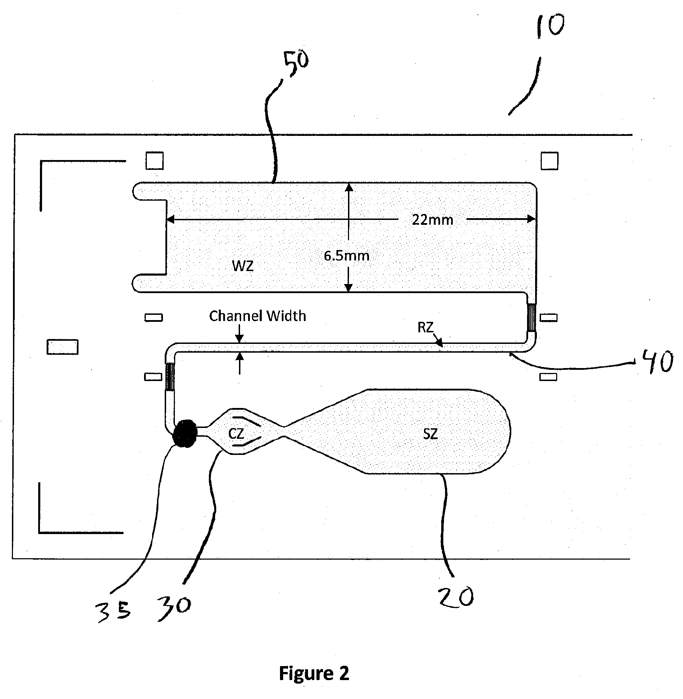

Optionally located in the fluid flow path, before or after the reagent zone and before the detection zone is a reagent addition zone. The reagent addition zone is shown as 35 in Figures 2 and 3. The reagent addition zone can allow addition of a reagent externally from the device. For example, the reagent addition zone may be used to add an interrupting reagent that may be used to wash the sample and other unbound components present in the fluid flow path into the wicking zone. In a preferred embodiment the reagent addition zone 35 is located after the reagent zone 30.

Downstream from the reagent zone is the detection zone 40 which is in fluid communication with the reagent zone. The detection zone 40 may include projections or micropillars such as those described above. As also noted above, these projections are preferably integrally molded into the substrate from an optical plastic material such as Zeonor, such as injection molding or embossing. As described above, the width of the flow path in the detection zone for conventionally sized devices is typically on the order of 2mm, which generally provides sufficient signal for the instrument to read even if the reagent plume does not cover the entire width of the detection zone.

The detection zone is where any detectable signal is read. In a preferred embodiment attached to the projections in the detection zone are capture elements. The capture elements can include binding partners for the conjugate or complexes containing the conjugate, as described above. For example, if the analyte is a specific protein, the conjugate may be an antibody that will specifically bind that protein coupled to a detection element such as a fluorescence probe. The capture element could then be another antibody that also specifically binds to that protein. In another example, if the marker or analyte is DNA, the capture molecule can be, but is not limited to, synthetic oligonucleotides, analogues thereof, or specific antibodies. Other suitable capture elements include antibodies, antibody fragments, aptamers, and nucleic acid sequences, specific for the analyte to be detected. A non-limiting example of a suitable capture element is a molecule that bears avidin functionality that would bind to a conjugate containing a biotin functionality. The detection zone can include multiple detection zones. The multiple detection zones can be used for assays that include one or more markers. In the event of multiple detection zones, the capture elements can include multiple capture elements, such as first and second capture elements. The conjugate can be pre-deposited on the assay device, such as by coating in the reagent zone. Similarly the capture elements can be pre-deposited on the assay device on the detection zone. Preferably, both the detection and capture elements are pre-deposited on the assay device, on the reaction zone and detection zone, respectively.

After the sample has been delivered to the sample zone, it will encounter the reagent zone. After the sample has flowed through and interacted with the reagent zone and optionally the reagent addition zone, the sample and a reagent plume will be contained in the fluid flow. The reagent plume can contain any of the reagent materials that have been dissolved in the reaction zone or those added through the reagent addition zone. The reagent plume can include the conjugate having both the detection element and binding partner, in which case it is often referred to as a conjugate plume. As noted throughout, one challenge facing the inventors was to keep the reagent plume as wide as possible as it enters the detection zone.

However, as described above, for smaller volume devices, such as those described above, simply downsizing a conventional sized device did not provide adequate signal. The inventors found that the width of the detection zone was too narrow to provide adequate signal. As a result, contrary to the need to reduce sample size, by reducing the volume of the device, the inventors found that it was necessary to maintain a relatively wide detection zone in the smaller volume device. Accordingly, in a preferred embodiment, the width of the detection zone flow path is greater than 0.5mm, preferably greater than 0.7mm, preferably greater than 0.8mm, more preferably greater than 0.9mm and most preferably around 1 mm.

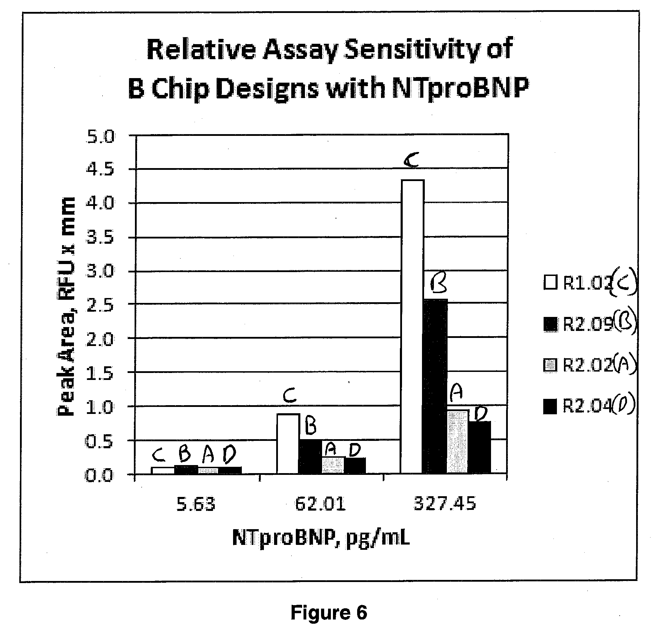

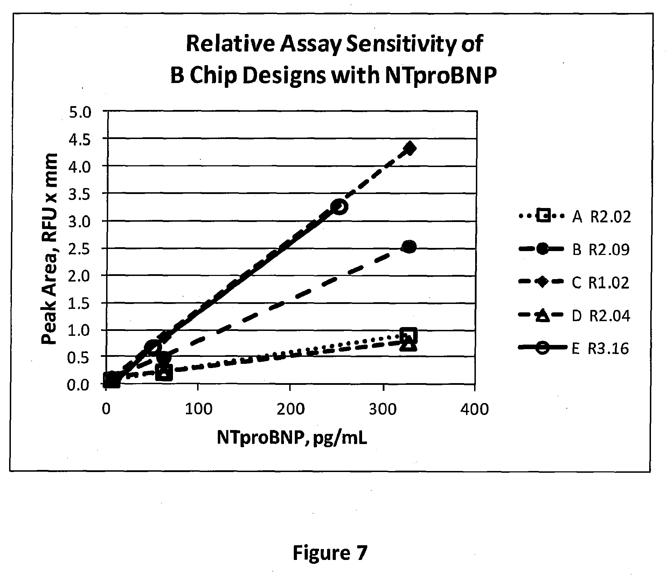

The increased signal made possible by the wider reagent plume and increased detection zone width is shown in the graphs in Figs. 6 and 7. Sample volumes of 15 microliters of serum were employed in testing chip designs R2.02, R2.04, R2.09 and R3.16. The R1.02 chip design was a control chip, intended for use with 200 microliters of whole blood. R1.02 chips were tested in this example with 45 microliters of serum. Bar and curve A (R2.02) is a miniaturized device having a single-reagent cell and a directly scaled down detection zone having a detection zone width of 0.5mm, whereas bar and curve B (R2.09) is a miniaturized device having dual reagent cell and a wider detection zone of 1 mm. Data for two additional device designs is also included for comparison. Curve and bar C (R1.02) is a conventionally sized assay device having a 200 uL whole blood sample volume, and curve and bar D (R2.04) is a single reagent cell device having a 1 mm detection zone width. Curve E (R3.16) includes dual reagent cells and a 1mm wide detection zone. As the results show, the assay device represented by B having the wider reagent plume and increased detection zone according to the present invention has significantly improved sensitivity compared to other miniaturized designs. Even more significant is the assay device represented by E having a sensitivity that is equivalent to a conventionally sized device, which was unexpectedly surprising given the approximately 10x greater amount of sample that is available to generate signal in a conventional device.

Together the increased reagent plume width and the flow path width through the detection zone width provide an assay device that provides an increased amount of signal in the detection zone due to signal being generated across a significant portion of the detection zone. This allows a greatly reduced sample size compared to conventionally sized assay devices. With the present invention, sample sizes on the order of ≤ 50 µl, ≤ 40 µl, ≤ 35 µl, ≤ 25 µl, and even ≤ 15 µl are possible. If whole blood is used, the size of the sample will be on the order of 25-50 µl, since a portion of the sample will be retained in the filter. If serum is used, the sample size can be less, e.g., on the order of 15 µl or less. A wash can also be used with whole blood. If a wash is used, the volume is one the order of 10 µl or less.

Downstream from the detection zone is a wicking zone in fluid communication with the detection zone. The wicking zone is an area of the assay device with the capacity of receiving liquid sample and any other material in the flow path, e.g., unbound reagents, wash fluids, etc. The wicking zone provides a capillary force to continue moving the liquid sample through and out of the detection zone. The wicking zone can include a porous material such as nitrocellulose or can be a non-porous structure such as the projections described herein. The wicking zone can also include non-capillary fluid driving means, such as using evaporative heating or a pump. Further details of wicking zones as used in assay devices according to the present invention can be found in patent publications US 2005/0042766 and US 2006/0239859,. Wicking zones are also described in copending patent application entitled "Controlling Fluid Flow Through An Assay Device" (Application No. 61/588772, Attorney Docket No. CDS 5112USPSP, first named inventor: James Kanaley), filed January 20. As described above, one disadvantage of miniaturizing the assay device is a reduced total assay flow time which reduces the contact time for interaction between reagent and sample in the reagent zone and the detection zone, such as binding in the reaction or detection zone. One preferred embodiment for increasing total assay flow time is to modify the wicking zone. As noted above, the wicking zone provides the capillary force to move the liquid through and out of the detection zone. How quickly or slowly this occurs can be controlled by altering the configuration of the wicking zone. One particularly preferred embodiment uses a wicking zone that is rectangular in shape and the longer side of the rectangle extends in the direction of flow. This reduces the pressure gradient in the assay device which decreases the flow rate of liquid sample compared to a wicking zone having equal length sides. Additional details of wicking zones can be found in copending patent application entitled "Controlling Fluid Flow Through An Assay Device" (Application No. 61/588772, Attorney Docket No. CDS 5112USPSP). Any methods to increase total assay flow time can be used in this aspect of the present invention and can include decreased flow path width, barriers in the flow path to slow down flow, etc.

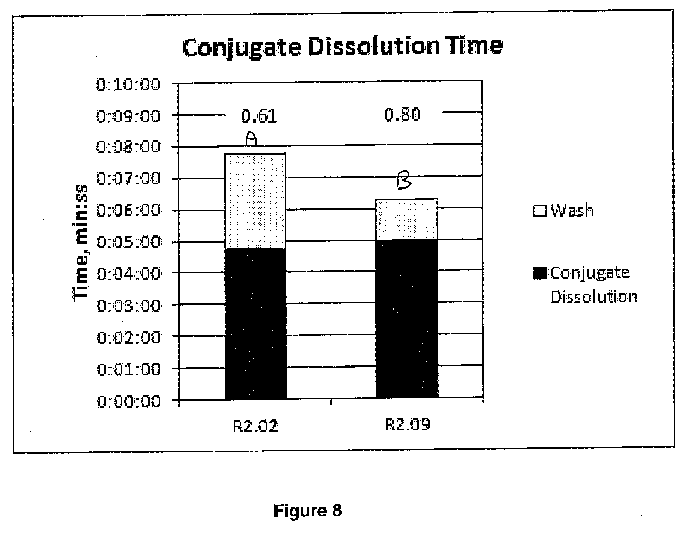

According to another aspect of the invention, the inventors have found that reagent dissolution time can be maximized by increasing the amount of reagent material in the reagent zone, such as by splitting the reagent material as described in the co-pending application entitled "Assay Device Having Multiple Reagent Cells" (described above) even though the total flow time is reduced compared to a conventionally sized assay device. More surprisingly, the inventors have found the amount of wash required can be minimized while still maintaining desired performance. Figure 8 shows a comparison of two assay devices with one (Bar A - R2.202) having a 0.5mm flow channel width and the other (Bar B - R2.09) having a 1.0 mm flow channel width. As Figure 8 shows, the assay device having the wider flow channel (Bar B) has a shorter total flow time (i.e., just over 6 minutes) compared to the assay device with a more narrow flow channel (Bar A) (i.e., flow time just under 8 minutes), as would be expected. However, the reagent dissolution time for the assay device depicted by Bar B is somewhat greater than the reagent dissolution time for the assay device depicted by Bar A. This is made possible by the surprising discovery that the ratio of wash to total flow time can be decreased while still maintaining adequate assay performance.

Preferably the entirety of the flow path including the sample addition zone, the detection zone and the wicking zone includes projections substantially vertical in relation to the substrate, and having a height, diameter and reciprocal spacing capable of creating lateral flow of the sample in the flow path.

In any of the above embodiments, the device is preferably a disposable assay device. The assay device may be contained in a housing for ease of handling and protection. If the assay device is contained in such a housing, the housing will preferably include a port for adding sample to the assay device.

The assay device of the present invention can be used with a device for reading (a reader) the result of an assay device performed on the assay of the present invention. The reader includes means for reading a signal emitted by, or reflected from the detection element, such as a photodetector, and means for computing the signal and displaying a result, such as microprocessor that may be included within an integrated reader or on a separate computer. Suitable readers are described for example in US 2007/0231883 and US Patent No. 7,416,700.

Such readers comprise a detector capable of reading a signal emitted from or reflected from at least one detection element present in a defined location of the assay device. The reading is preferably chosen from the detection and/or quantification of color, fluorescence, radioactivity or enzymatic activity.

Another aspect of the invention is directed to a method of performing an assay on a liquid sample for the detection of one or more analytes of interest as defined in the claims. A liquid sample suspected of containing the analyte(s) of interest is deposited onto the sample addition zone of the assay device, such as through a port in the housing of the device, or by touching off a finger directly onto the sample addition zone in the case of a fingerstick blood draw. The sample moves by capillary action through an optional filter, and into the reagent zone where it dissolves the reagent material. In a preferred embodiment, the sample is reacted with a detection element in the case of a sandwich type assay, either directly or indirectly, such as through an antibody. The sample flows away from the reagent zone having a dissolved reagent plume as in flows into the detection zone.

Next the sample moves by capillary action into the detection zone, where the reagent plume extends substantially the width of the detection zone. The reagent plume extends at least 80% and more preferably at least 90% of the width of the detection zone. In the detection zone, a signal representative of the analyte(s) or control is produced. In a preferred embodiment the sample or the one or more reagents having a detection element is captured having in the detection zone, such as by antibodies on the surface of the detection zone and a signal representative of the presence or concentration of the analyte(s) or control(s) is produced. The reader or detection instrument as described above is then used to read the signal that is produced by the detection zone, such as by reading a signal that is produced by the detection element to determine the presence or concentration of the analyte(s) or control(s). The sample moves from the detection zone and into the wicking zone. The reader may read the signal immediately or a short time after the sample has moved through the detection zone. Also, one or more washes may follow the sample through the device to wash any unbound detection element away from the detection zone.

The method, assay device, and reader according to an embodiment of the invention have many advantages, mainly related to the improved reaction kinetics of the immunochemical reactions and the increased sensitivity of the assay.

It is to be understood that this invention is not limited to the particular embodiments shown here. The following examples are provided for illustrative purposes and are not intended to limit the scope of the invention since the scope of the present invention is limited only by the appended claims.

### Examples

### Example 1

Plastic substrate chips made of Zeonor (Zeon, Japan) having oxidized dextran on the surface for covalently immobilization of proteins via Schiff base coupling were used. Fluorescently labeled Anti-NT-proBNP monoclonal antibody was deposited and dried to create a reagent zone. Anti-NT-proBNP monoclonal antibody was deposited and dried to create a detection zone. A small amount of Triton X-45 was deposited on the device to increase wettability of the sample for better capillary flow. Serum spiked with NT-proBNP was added to the sample zone of the device and the capillary action of the micropillar array distributed the sample through the flow channel into the wicking zone. Sample volumes of 15 microliters were employed on low-volume chip designs R2.02, R2.04, R2.09 and R3.16. The R1.02 chip design was a control chip, intended for use with 200 microliters of whole blood. R1.02 chips were tested in this example with 45 microliters of serum. A typical assay time was about 10 minutes. The signal intensities from the fluorescently labeled complexes in the detection zone were recorded in a prototype line-illuminating fluorescence scanner. The results are shown in Figures 6-8 described above.

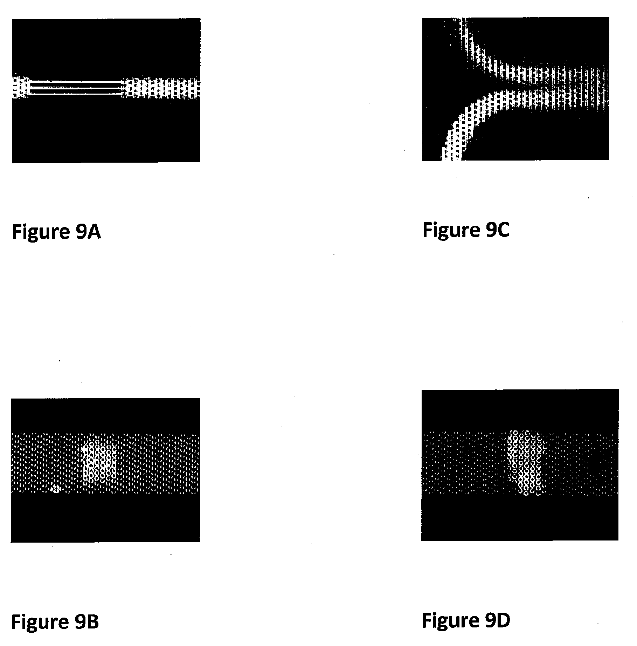

Figure 9A shows the width of the reagent plume using one reagent cell. Figure 9B shows the width of the signal generated in the detection zone. Figure 9C shows the width of the reagent plume using two reagent cells according to the present invention. Figures 9A and 9C clearly show the multiple reagent cells provides a significantly wider plume than a single reagent cell. Figures 9B and 9D show that the multiple reagent plume provides a wider signal generated in the detection zone which translates into more signal being read by the instrument.

### Example 2

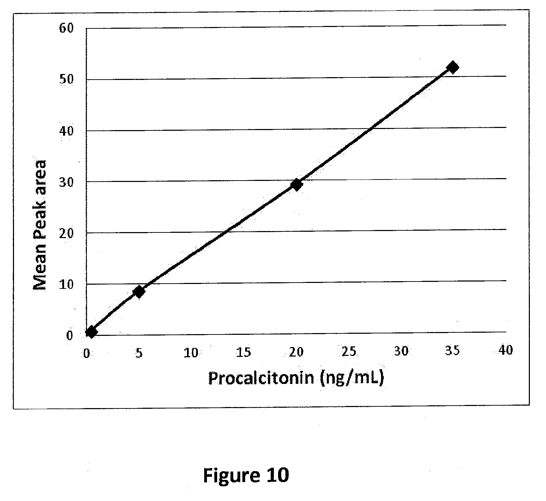

Plastic substrate assay devices made of Zeonor (Zeon, Japan) having dual reagent cells and a 1 mm detection zone width, and having oxidized dextran on the surface for covalently immobilization of proteins via Schiff base coupling were used. Fluorescently labeled anti-procalcitonin monoclonal antibody was deposited and dried to create a reagent zone. Anti-procalcitonin monoclonal antibody was deposited and dried to create a detection zone. A small amount of Triton X-45 was deposited on the device to increase wettability of the sample for better capillary flow. In this example 25 microliters of whole blood containing procalcitonin was applied to a filter in contact with the sample addition zone of the assay device. Plasma is transferred from the filter into the sample addition zone and then moves by capillary force through the flow path to the wicking zone. The fluid flow was monitored by visual inspection and 10 microliters of a wash fluid containing 0.01 M phosphate buffer, 0.0027 M potassium chloride, 0.137 M sodium chloride, 1% bovine serum albumin and 0.1% triton X-100 was added to the reagent addition zone when the fluid flow front filled 20% of the wicking zone. The assay device was inserted into a fluorescent reader immediately after the wicking zone was determined to be completely filled. The fluorescent signal within the detection zone was measured and the peak area under the response curve was determined for each sample. Whole blood samples were collected fresh from normal donors in lithium heparin tubes. A concentrated serum sample containing 10 ug/mL procalcitonin was added to aliquots of whole blood to create samples containing 0, 0.4, 5, 20 and 35 ng/mL procalcitonin. Figure 10 plots the mean peak area of five replicate results for each sample versus the procalcitonin concentration. As Figure 10 demonstrates, using a small sample size (i.e., 25 µL whole blood/10 µL wash) provides satisfactory results over a wide range of analyte concentrations.

### Example 3

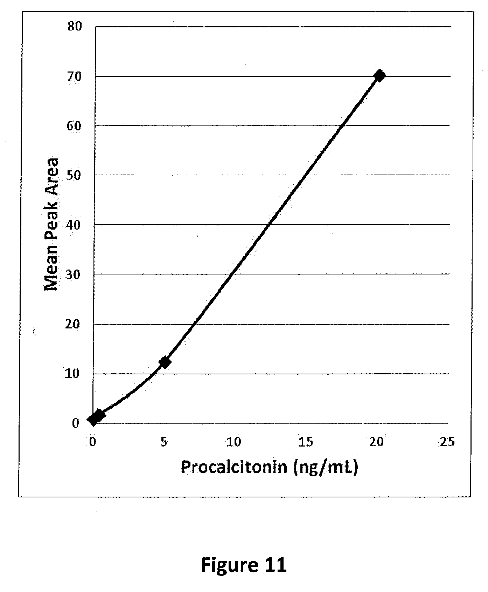

Plastic substrate assay devices made of Zeonor (Zeon, Japan) having dual reagent cells and a 1 mm detection zone width, and having oxidized dextran on the surface for covalently immobilization of proteins via Schiff base coupling were used. Fluorescently labeled anti- procalcitonin monoclonal antibody was deposited and dried to create a reagent zone. Anti- procalcitonin monoclonal antibody was deposited and dried to create a detection zone. A small amount of Triton X-45 was deposited on the device to increase wettability of the sample for better capillary flow. In this example thirty five microliters of whole blood containing procalcitonin was applied to a filter in contact with the sample addition zone of the assay device. Plasma is transferred from the filter into the sample addition zone then moves by capillary force through the flow path to the wicking zone. The fluid flow was monitored by visual inspection and inserted into the fluorescent reader immediately after the wicking zone was determined to be completely filled. The fluorescent signal within the detection zone was measured and the peak area under the response curve was determined for each sample. Whole blood samples were collected fresh from normal donors in EDTA tubes. A concentrated serum sample of 10 ug/mL procalcitonin was added to aliquots of whole blood to create samples containing 0, 0.4, 5, and 20 ng/mL procalcitonin. Figure 11 plots the mean peak area of three replicate results for each sample versus the procalcitonin concentration. As Figure 11 demonstrates, using a small sample size (i.e., 35 µL whole blood) provides satisfactory results over a wide range of analyte concentrations.

## Claims

1. An assay device comprising:
a liquid sample zone;
a reagent zone downstream and in fluid communication with the sample addition zone containing a reagent material;
a detection zone in fluid communication with the reagent zone, wherein the detection zone has a substrate and projections which extend vertically from the substrate, wherein the projections have a height, cross-section and a distance between one another that defines a capillary space between the projections capable of generating capillary flow parallel to the substrate surface;
wherein
the reagent zone comprises at least two reagent cells containing the reagent material and arranged in the reagent zone such that each reagent cell experiences the same flow conditions of sample from the sample addition zone,
wherein the reagent cells divide the sample flow from the sample addition zone into multiple flow streams, and
one or more flow control elements disposed downstream from the reagent zone which combine the multiple flow streams into fewer flow streams; and wherein
the device is capable of creating a reagent plume in the detection zone that comprises liquid sample and dissolved reagent, wherein the width of the reagent plume extends across at least 80% of the width of the detection zone; and
a wicking zone in fluid communication with the detection zone having a capacity to receive liquid sample flowing from the detection zone.

2. An assay device as claimed in claim 1, wherein the reagent material is a labeled reagent material, the device is capable of creating a reagent plume that comprises dissolved labeled reagent and the detection zone has capture elements bound thereto.

3. An assay device as claimed in claim 1, wherein the device is capable of creating a reagent plume that extends across at least 90% of the width of the detection zone.

4. An assay device as claimed in claim 1, wherein the width of the detection zone is 0.7 to 1.5 mm.

5. An assay device as claimed in claim 1, wherein the total area of the assay device is ≤ 900 mm².

6. An assay device as claimed in claim 1, wherein the assay device is rectangular and the dimensions of each side are ≤ 30mm.

7. An assay device according to claim 1, wherein the assay device is capable of using a sample size of ≤ 50 µl.

8. An assay device according to claim 1, wherein the assay device is capable of using a sample size of ≤ 25 µl.

9. An assay device according to claim 1, wherein the total area of the assay device is ≤ 900 mm2, preferably ≤ 700 mm2.

10. A method for performing an assay on a liquid sample for the detection of one or more analytes of interest, the method comprising the steps of:
providing a liquid sample addition zone for receiving the liquid sample;
providing a reagent zone in fluid communication with the sample addition zone, said reagent zone comprising at least two reagent cells containing the reagent material and arranged in the reagent zone such that each reagent cell experiences the same flow conditions of sample from said sample addition zone,
wherein the reagent cells divide the sample flow from the sample addition zone into multiple flow streams; and
providing a detection zone in fluid communication with the reagent zone having capture elements bound thereto, wherein the detection zone has a substrate and projections which extend vertically from the substrate, wherein the projections have a height, cross-section and a distance between one another that defines a capillary space between the projections capable of generating capillary flow parallel to the substrate surface;
providing a wicking zone in fluid communication with the detection zone having a capacity to receive liquid sample flowing from the detection zone;
dispensing the sample onto the sample zone, whereby the sample flows by capillary action from the sample zone and into the reagent zone, where the sample dissolves the reagent material and forms a reagent plume that comprises liquid sample and dissolved reagent;
flowing the sample/reagent plume by capillary action into the detection zone, wherein the width of the reagent plume extends across at least 80% of the width of the detection zone, wherein a signal representative of the presence of concentration of analyte(s) or control(s) is produced; and
reading the signal that is produced in the detection zone to determine the presence or concentration of the one or more analyte.

11. A method as claimed in claim 10, wherein reagent material comprises a detection element, the detection zone has capture elements bound thereto, and at least a portion of the dissolved reagent material reacts with analyte in the sample.

12. A method as claimed in claim 10, wherein the analyte(s) or the one or more reagents having a detection element is captured by capture elements in the detection zone, and a signal representative of the presence or concentration of the analyte(s) or control(s) is detected.

## Patentansprüche

1. Eine Analysevorrichtung, aufweisend:
eine Flüssigkeitsprobenzone;
eine ein Reagenzmaterial enthaltende Reagenzzone stromabwärts von der Probenzugabezone gelegen und in Fluidverbindung mit dieser;
eine Detektionszone in Fluidverbindung mit der Reagenzzone, wobei die Detektionszone ein Substrat und sich vertikal von dem Substrat erstreckende Vorsprünge hat, wobei die Vorsprünge eine Höhe, einen Querschnitt und einen Abstand voneinander haben, der einen Kapillarraum zwischen den Vorsprüngen definiert, der in der Lage ist einen kapillaren Fluss parallel zu der Substratoberfläche zu erzeugen;
wobei
die Reagenzzone wenigstens zwei Reagenzzellen aufweist, die das Reagenzmaterial enthalten und derart in der Reagenzzone angeordnet sind, dass jede Reagenzzelle dieselben Flussbedingungen von einer Probe aus der Probenzugabezone erfährt,
wobei die Reagenzzellen den Probenfluss aus der Probenzugabezone in mehrere Flussströme unterteilen, und
ein oder mehrere Flusssteuerungselemente, die stromabwärts von der Reagenzzone gelegen sind, die die mehreren Flussströme in weniger Flussströme vereinen; und
wobei
die Vorrichtung in der Lage ist, eine Reagenzfahne in der Detektionszone zu kreieren, die eine Flüssigkeitsprobe und ein gelöstes Reagenz aufweist, wobei sich die Breite der Reagenzfahne über wenigstens 80% der Breite der Detektionszone erstreckt; und
eine Dochtwirkungszone in Fluidverbindung mit der Detektionszone mit einer Kapazität, um eine aus der Detektionszone herausströmende Flüssigkeit zu empfangen.

2. Eine Analysevorrichtung wie in Anspruch 1 beansprucht, wobei das Reagenzmaterial ein bezetteltes Reagenzmaterial ist, die Vorrichtung in der Lage ist, eine Reagenzfahne zu kreieren, die ein gelöstes bezetteltes Reagenz aufweist und die Detektionszone daran gebundene Fangelemente hat.

3. Eine Analysevorrichtung wie in Anspruch 1 beansprucht, wobei die Vorrichtung in der Lage ist eine Reagenzfahne zu kreieren, die sich über wenigstens 90% der Breite der Detektionszone erstreckt.

4. Eine Analysevorrichtung wie in Anspruch 1 beansprucht, wobei die Breite der Detektionszone 0,7 bis 1,5 mm ist.

5. Eine Analysevorrichtung wie in Anspruch 1 beansprucht, wobei die Gesamtfläche der Analysevorrichtung ≤ 900 mm² ist.

6. Eine Analysevorrichtung wie in Anspruch 1 beansprucht, wobei die Analysevorrichtung rechteckig ist und die Abmessungen jeder Seite ≤ 30 mm sind.

7. Eine Analysevorrichtung wie in Anspruch 1 beansprucht, wobei die Analysevorrichtung in der Lage ist eine Probengröße von ≤ 50 µl zu verwenden.

8. Eine Analysevorrichtung wie in Anspruch 1 beansprucht, wobei die Analysevorrichtung in der Lage ist eine Probengröße von ≤ 25 µl zu verwenden.

9. Eine Analysevorrichtung wie in Anspruch 1 beansprucht, wobei die Gesamtfläche der Analysevorrichtung ≤ 900 mm², bevorzugt ≤ 700 mm² ist.

10. Ein Verfahren zum Durchführen einer Analyse bei einer Flüssigkeitsprobe zur Detektierung von einem oder mehreren interessanten Analyten, das Verfahren aufweisend die Schritte von:
Bereitstellen einer Flüssigkeitsprobenzugabezone zum Empfangen der Flüssigkeitsprobe;
Bereitstellen einer Reagenzzone in Fluidverbindung mit der Probenzugabezone, wobei die besagte Reagenzzone wenigstens zwei Reagenzzellen aufweist, die das Reagenzmaterial enthalten und derart in der Reagenzzone angeordnet sind, dass jede Reagenzzelle dieselben Flussbedingungen von einer Probe aus der besagten Probenzugabezone erfährt,
wobei die Reagenzzellen den Probenfluss aus der Probenzugabezone in mehrere Flussströme unterteilen; und
Bereitstellen einer Detektionszone in Fluidverbindung mit der Reagenzzone mit daran gebundenen Fangelementen, wobei die Detektionszone ein Substrat und sich vertikal von dem Substrat erstreckende Vorsprünge hat, wobei die Vorsprünge eine Höhe, einen Querschnitt und einen Abstand voneinander haben, der einen Kapillarraum zwischen den Vorsprüngen definiert, der in der Lage ist einen kapillaren Fluss parallel zu der Substratoberfläche zu erzeugen;
Bereitstellen einer Dochtwirkungszone in Fluidverbindung mit der Detektionszone mit einer Kapazität, um eine aus der Detektionszone herausströmende Flüssigkeit zu empfangen;
Dispensieren der Probe auf der Probenzone, wobei die Probe durch Kapillarwirkung von der Probenzone in die Reagenzzone strömt, wo die Probe das Reagenzmaterial löst und eine Reagenzfahne bildet, die eine Flüssigkeitsprobe und ein gelöstes Reagenz aufweist;
Strömen der Proben/Reagenz-Fahne durch Kapillarwirkung in die Detektionszone, wobei sich die Breite der Reagenzfahne über wenigstens 80% der Breite der Detektionszone erstreckt, wobei ein Signal, das repräsentativ für die Anwesenheit einer Konzentration von einem Analyt/Analyten oder einer Kontrolle/n ist, erzeugt wird; und
Lesen des in der Detektionszone erzeugten Signals, um die Anwesenheit oder Konzentration von dem einen Analyt oder den mehreren Analyten zu bestimmen.

11. Ein Verfahren wie in Anspruch 10 beansprucht, wobei das Reagenzmaterial ein Detektionselement aufweist, die Detektionszone Fangelemente daran gebunden hat und wenigstens ein Teil des gelösten Reagenzmaterials mit einem Analyten in der Probe reagiert.

12. Ein Verfahren wie in Anspruch 10 beansprucht, wobei das Analyt/ die Analyten oder das eine Reagenz oder die mehreren Reagenzien mit einem Detektionselement durch Fangelemente in der Detektionszone gefangen ist/sind und ein Signal, das repräsentativ für die Anwesenheit oder Konzentration des Analyts/ der Analyten oder der Kontrolle/n ist, detektiert wird.

## Revendications

1. Dispositif de dosage comprenant :
une zone d'échantillon de liquide ;
une zone de réactif en aval et en communication fluidique avec la zone d'addition d'échantillon contenant un matériau réactif ;
une zone de détection en communication fluidique avec la zone de réactif, dans lequel la zone de détection a un substrat et des saillies qui s'étendent verticalement depuis le substrat, dans lequel les saillies ont une hauteur, une section transversale et une distance entre elles qui définissent un espace capillaire entre les saillies capable de générer un écoulement capillaire parallèle à la surface du substrat ; dans lequel
la zone de réactif comprend au moins deux cellules de réactif contenant le matériau réactif et agencées dans la zone de réactif de sorte que chaque cellule de réactif subisse les mêmes conditions d'écoulement d'échantillon provenant de la zone d'addition d'échantillon,
dans lequel les cellules de réactif divisent l'écoulement d'échantillon provenant de la zone d'addition d'échantillon en de multiples courants d'écoulement, et
un ou plusieurs éléments de commande d'écoulement disposés en aval de la zone de réactif qui combinent les multiples courants d'écoulement en un nombre moindre de courants d'écoulment ; et
dans lequel :
le dispositif est capable de créer une colonne de réactif dans la zone de détection qui comprend un échantillon de liquide et un réactif dissous, dans lequel la largeur de la colonne de réactif s'étend à travers au moins 80 % de la largeur de la zone de détection ; et
une zone de mèche en communication fluidique avec la zone de détection ayant une capacité à recevoir un échantillon de liquide s'écoulant depuis la zone de détection.

2. Dispositif de dosage selon la revendication 1, dans lequel le matériau réactif est un matériau réactif marqué, le dispositif est capable de créer une colonne de réactif qui comprend du réactif marqué dissous et la zone de détection présente des éléments de capture qui y sont liés.

3. Dispositif de dosage selon la revendication 1, dans lequel le dispositif est capable de créer une colonne de réactif qui s'étend à travers au moins 90 % de la largeur de la zone de détection.

4. Dispositif de dosage selon la revendication 1, dans lequel la largeur de la zone de détection est de 0,7 à 1,5 mm.

5. Dispositif de dosage selon la revendication 1, dans lequel la surface totale du dispositif de dosage est ≤ 900 mm².

6. Dispositif de dosage selon la revendication 1, dans lequel le dispositif de dosage est rectangulaire et les dimensions de chaque côté sont ≤ 30 mm.

7. Dispositif de dosage selon la revendication 1, dans lequel le dispositif de dosage est capable d'utiliser une taille d'échantillon ≤ 50 µL.

8. Dispositif de dosage selon la revendication 1, dans lequel le dispositif de dosage est capable d'utiliser une taille d'échantillon ≤ 25 µL.

9. Dispositif de dosage selon la revendication 1, dans lequel la surface totale du dispositif de dosage est ≤ 900 mm², de préférence ≤ 700 mm².

10. Procédé de réalisation d'un dosage sur un échantillon de liquide pour la détection d'un ou plusieurs analytes d'intérêt, le procédé comprenant les étapes consistant à :
fournir une zone d'addition d'échantillon de liquide pour recevoir l'échantillon de liquide ;
fournir une zone de réactif en communication fluidique avec la zone d'addition d'échantillon, ladite zone de réactif comprenant au moins deux cellules de réactif contenant le matériau réactif et agencées dans la zone de réactif de sorte que chaque cellule de réactif subisse les mêmes conditions d'écoulement d'échantillon provenant de ladite zone d'addition d'échantillon,
dans lequel les cellules de réactifs divisent l'écoulement d'échantillon provenant de la zone d'addition d'échantillon en de multiples courants d'écoulement ; et
fournir une zone de détection en communication fluidique avec la zone de réactif ayant des éléments de capture qui y sont liés, dans lequel la zone de détection a un substrat et des saillies qui s'étendent verticalement depuis le substrat, dans lequel les saillies ont une hauteur, une coupe transversale et une distance entre elles qui définissent un espace capillaire entre les saillies capable de générer un écoulement capillaire parallèle à la surface du substrat ;
fournir une zone de mèche en communication fluidique avec la zone de détection ayant une capacité à recevoir un échantillon de liquide s'écoulant de la zone de détection ;
distribuer l'échantillon sur la zone d'échantillon, l'échantillon s'écoulant ainsi par action capillaire depuis la zone d'échantillon et dans la zone de réactif, où l'échantillon dissout le matériau réactif et forme une colonne de réactif qui comprend un échantillon de liquide et un réactif dissous ;
faire couler l'échantillon/la colonne de réactif par action capillaire dans la zone de détection, dans lequel la largeur de la colonne de réactif s'étend à travers au moins 80 % de la largeur de la zone de détection, dans lequel un signal représentatif de la présence ou de la concentration d'analyte(s) ou de témoin(s) est produit ; et
lire le signal qui est produit dans la zone de détection pour déterminer la présence ou la concentration des un ou plusieurs analytes.

11. Procédé selon la revendication 10, dans lequel le matériau réactif comprend un élément de détection, la zone de détection présente des éléments de capture qui y sont liés et au moins une partie du matériau réactif dissous réagit avec un analyte dans l'échantillon.

12. Procédé selon la revendication 10, dans lequel le ou les analytes ou les un ou plusieurs réactifs ayant un élément de détection sont capturés par des éléments de capture de la zone de détection et un signal représentatif de la présence ou de la concentration du ou des analytes ou du ou des témoins est détecté.
